# EUROPEAN PATENT APPLICATION

(11) **EP 1 642 566 A1**
(43) Date of publication of application: **05.04.2006**
(21) Application number: 04292331.8
(22) Date of filing: 30.09.2004
(51) Int. Cl.: A61K 8/49, A61K 8/67, A61K 8/97, A61Q 19/08

(54) **Retinoid-containing compositions having reduced irritation effect**

(71) Applicant: Johnson & Johnson Consumer France SAS, 92787 Issy les Moulineaux Cedex 9 (FR)
(72) Inventor: Dupressoir, Carole, 78300 Poissy (FR); Oddos, Thierry, 92190 Meudon (FR); Michelon, Véronique, 7600 Ruen (FR)
(74) Representative: Weber-Bruls, Dorothée

(57) **Abstract**

The present invention features a composition including (i) a retinoid, and (ii) an interleukin-8 inhibitor. The composition provides reduced irritation to human skin.

## Description

### Field of invention

The present invention relates to compositions comprising a retinoid, and an interleukin-8 inhibitor.

### Background of the invention

It has become desirable to include various oxygen-labile active agents in topical skin care compositions in order to provide a cosmetic/therapeutic benefit, e.g., to the skin and hair. Examples of such active agents include, but are not limited to, vitamins such as vitamin C, vitamin E, vitamin K, and vitamin A, and retinoids such as retinol. Other oxygen-labile active agents include ubiquinone and hydroquinone.

Retinoids, in particular retinol, are commonly used in topical skin care compositions to reduce the appearance of aging. However, retinoids may also cause irritation and inflammation characterized by redness, dryness, scaling and stinging. In connection with this, it is known that retinol induces the release by the skin of the inflammation mediator interleukin-8. The release may be delayed by 48 to 72 hours.

Therefore, there is a need for an improved composition comprising a retinoid that induces less interleukin-8 production. Applicants have discovered that certain compounds are capable, upon combination with retinoids, of specifically reducing the amount interleukin-8 produced by the epidermis, and therefore reducing the irritation caused by such compositions.

### Summary of the invention

The present invention provides a composition comprising: (a) a retinoid, and (b) an interleukin-8 inhibitor, wherein said composition provides at least about a 40 percent reduction in interleukin-8 produced by human epidermis after contact with said composition compared with a control composition comprising said retinoid but not said interleukin-8 inhibitor.
The present invention also provides a composition comprising: (a) about 0.001 to about 1 weight percent of retinol, and (b) about 0.0005 to about 5 weight percent of an interleukin-8 inhibitor.

Other features and advantages of the present invention will be apparent from the detailed description of the invention and from the claims.

### Detailed description of the invention

It is believed that one skilled in the art can, based upon the description herein, utilize the present invention to its fullest extent. The following specific embodiments are to be construed merely to be illustrative and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all isomers are included for compounds (e.g., tocopherol) where no specific isomer is indicated.

As used herein, an active agent is a compound that offers a cosmetic, pharmaceutical, or therapeutic benefit when applied to the skin of a mammal (e.g., when topically administered to the skin or hair of a human).

Suitable active agents include as component (a), for example, retinoids.

Examples of retinoids include retinol, retinal, and retinoic acid.

The amount of retinoid in the composition will depend upon the retinoid used and the desired therapeutic/cosmetic effect, and typically will range from about 0.001% to about 20% (e.g., from about 0.01% to about 10%), by weight, of the composition. In one embodiment the composition comprises from about 0.001 % to about 1% (e.g., from about 0.01% to about 0.5%), by weight, of a retinoid such as retinol.

The composition also comprises an interleukin-8 inhibitor, that is, a compound that, when combined with retinoid, results in a reduction in the level of interleukin-8 released by epidermis that has been contacted with the composition.

In one embodiment, the interleukin-8 inhibitor comprises an oat extract. More particularly, the oat extract comprises an avenanthramide or avenanthramide derivatives. Avenanthramides are naturally occurring anthranilic acid derivatives found in oats. See *Oats: Chemistry and Technology* (1986) Ed. Webster AACC, St. Paul, MN, pp.227-86. Avenanthramides have the general formula:

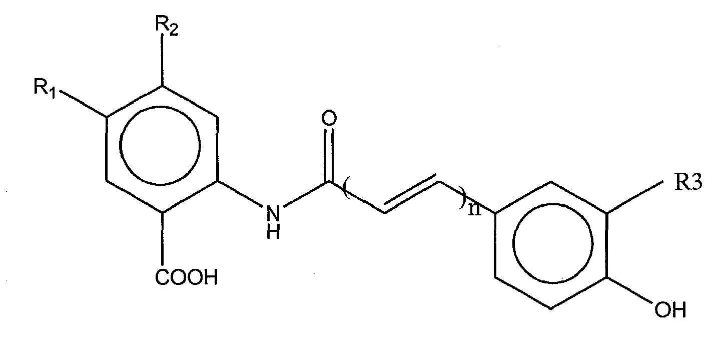

Wherein:
R1 = OH or H
R2 = H
R3 = H or OR wherein R is an alkyl group such as methyl, ethyl, propyl, butyl, pentyl or the like (including all isomeric forms), and
n = 1 or 2

One particularly suitable avenanthramide-containing interleukin-8 inhibitor is Dragocalm®, commercially available from Symrise.

In one embodiment, the interleukin-8 inhibitor comprises avenanthramide or an avenanthramide derivative.

In another embodiment, the interleukin-8 inhibitor comprises ectoine (1,4,5,6-tetrahydro-2-methyl-4-pyrimidine carboxylic acid) or an ectoine derivative, such as hydroxyectoine. Ectoine and ectoine derivatives are low molecular weight, cyclic amino acid derivatives that can be obtained from microorganisms and have the following structure:

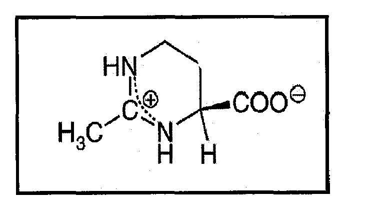

One particularly suitable ectoine-containing interleukin-8 inhibitor is commercially available from Merck under the name Rona Care® Ectoine.

Typically, about 0.0005 to about 5 weight percent of interleukin-8 inhibitor is present in the composition.

The compositions of the present invention are suitable for topical application to skin. The compositions may be made into a wide variety of product types that include but are not limited to lotions, creams, gels, sticks, sprays, ointments, shampoos, pastes, mousses, and cosmetics. These product types may comprise several types of cosmetically acceptable carrier systems including, but not limited to solutions, emulsions, gels, solids and liposomes.

What is meant by "cosmetically acceptable carrier" is a carrier that is capable of having the retinoid and the interleukin-8 dispersed or dissolved therein, and of possessing acceptable safety properties (e.g., irritation and sensitization characteristics).

Compositions of the present invention formulated as solutions typically include an aqueous (e.g., water) or organic solvent (e.g., from about 80% to about 99.99% or from about 90% to about 99% of an acceptable aqueous or organic solvent). Examples of suitable organic solvents include: propylene glycol, polyethylene glycol (200-600), polypropylene glycol (425-2025), glycerol, 1,2,4-butanetriol, sorbitol esters, 1,2,6-hexanetriol, ethanol, butylenes glycol, and mixtures thereof.

Compositions of the invention formulated as a solutions may comprise one or more emollients. Such compositions typically contain from about 2% to about 50% of a an emollient(s). As used herein, "emollients" refer to materials used for the prevention or relief of dryness, as well as for the protection of the skin.
A lotion can be made from a solution carrier system. Lotions typically comprise from about 1% to about 20% (e.g., from about 5% to about 10%) of an emollient(s) and from about 50% to about 90% (e.g., from about 60% to about 80%) of water.

The composition may also be formulated with a solution carrier system as a cream. A cream typically comprises from about 5% to about 50% (e.g., from about 10% to about 20%) of an emollient(s) and from about 45% to about 85% (e.g., from about 50% to about 75%) of water.

Yet another type of product that may be formulated according to the invention from a solution carrier system is an ointment. An ointment may comprise a simple base of animal or vegetable oils or semi-solid hydrocarbons (oleaginous, absorbent, emulsion and water soluble ointment bases). Ointments may also comprise absorption ointment bases that absorb water to form emulsions. Ointment carriers may also be water-soluble. An ointment may comprise from about 2% to about 10% of an emollient(s) plus from about 0.1% to about 2% of a thickening agent(s).

If the carrier is formulated as an emulsion, typically from about 1% to about 10% (e.g., from about 2% to about 5%) of the carrier system comprises an emulsifier(s). Emulsifiers may be nonionic, anionic or cationic.

Lotions and creams can be formulated as emulsions. Typically such lotions comprise from 0.5% to about 5% of an emulsifier(s). Such creams would typically comprise from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type, are well-known in the cosmetic art and are useful in the subject invention. Multiphase emulsion compositions, such as the water-in-oil-in-water type, are also useful in the subject invention. In general, such single or multiphase emulsions contain water, emollients, and emulsifiers as essential ingredients.

The compositions useful in the subject invention may contain, in addition to the aforementioned components, a wide variety of additional oil-soluble materials and/or water-soluble materials conventionally used in topical compositions, at their art-established levels. Various water-soluble materials may also be present in the compositions useful in the subject invention. These include humectants, proteins and polypeptides, preservatives and an alkaline agent. In addition, the topical compositions useful herein can contain conventional cosmetic adjuvants, such as dyes, opacifiers (e.g., titanium dioxide), filters, pigments and perfumes.

The compositions of the present invention can be topically applied to the skin or hair of a mammal (e.g., by the direct laying on or spreading of the composition on the skin or hair of a human). Depending on the selection of the active agent (e.g., the retinoid or other active agents), the compositions can be used to treat a number of skin and hair disorders such as but not limited to acne, mottled hyperpigmentation, age spots, wrinkles, fine lines, cellulite, and other visible signs of aging (whether due to photoaging or chronoaging).

The composition of the invention and formulations and products containing such composition may be prepared using methodology that is well known by an artisan of ordinary skill.

Advantageously, the composition of the invention provides at least about a 40 percent, preferably at least about a 50 percent reduction in interleukin-8 produced by human epidermis after contact with said composition compared with a control composition comprising said retinoid but not said interleukin-8 inhibitor. Such a control composition contains the same ingredients in the same amounts, except for the interleukin-8 inhibitor. Accordingly, due to the presence of the interleukin-8 inhibitor, the level of interleukin-8 produced by the skin is decreased, thereby also decreasing the level of inflammation caused by application of the composition.

The following examples further illustrate the invention.

### Example 1

Samples of reconstituted epidermis were topically applied with gel/emulsions containing retinol (0.075 wt.%) and a series of test ingredients. The release of interleukin-8 (IL8) was measured in the culture medium after 48 hours by an ELISA method. No significant loss of viability was evidenced. No release of interleukin-1 (IL1) was observed.

Nine different test ingredients were evaluated for their potential to decrease the retinol-induced release of IL8. The results are shown in Table 1 below. Among the test ingredients only Dragocalm® at 3.5 wt.% (0.00035 wt.% active matter) and ectoin 1 wt.% showed an inhibitory activity on retinol induced IL8 release as they were able to decrease IL8 release by 44% and 55% respectively.

**Table 1**

| | % Reduction of Interleukin-8 compared with Control + Retinol |
|---|---|
| Control + Retinol 0.075 wt.% | - |
| + Stearyl Glycirrhezinate 0.1 wt.% | 26 |
| + Dipotassium Glycirrhezinate 0.2 wt.% | -29 |
| + Epaline (Zea Mays) 3.5 wt.% | 2 |
| + Alpha Bisabolol 0.05 wt.% | -4 |
| + Dragocalm® (avenanthramides) 3.5 wt.% | -44* |
| + Rona Care® Ectoin 1 wt.% | -55 |
| + Calmiskin (Pepermint extract) 3.5 wt.% | -15 |

| | |
|---|---|
| * Significant difference versus Control + Retinol 0.075 wt.% | |

### Example 2

Samples of reconstituted epidermis were topically applied with gel/emulsions containing retinol (0.075 wt.%) and one of the following test ingredients: Dragocalm® (at 1.5 wt.% and 3.5 wt.%), dipotassium glycyrrhenzinate (at 0.5 wt.%), Rona Care® ectoine (at 0.1 wt.%, 0.25 wt.%), and a willow herb (0.1 wt.%)/oat straw (0.1 wt.%) extract combination. The release of IL8 was measured in each culture medium after 48 hours and 96 hours by an ELISA method. No significant loss of viability was evidenced. No release of IL1 was observed.

Results are summarized in Table 2. The presence of Dragocalm®, at concentrations of 1.5 wt.% and 3.5 wt.% (0.0001 wt.% to 0.00035 wt.% of active matter), markedly reduced IL8 release by 56% and 78% respectively. Ectoine also decreased IL8 release. Ectoine showed a similar activity at 0.25 wt.% (60% of inhibition), and no activity at 0.1 wt.%. Dipotassium glycyrrhenzinate did show an inhibitory activity at 0.5 wt.% (63% of inhibition). Finally willow herb/oat straw combo did not show any activity.

**Table 2**

| Test products | % reduction of IL8 release Control + retinol |
|---|---|
| Control + retinol 0.075 wt.% | - |
| + Dragocalm® 1.5 wt.% | -56 |
| + Dragocalm® 3.5 wt.% | -78 |
| + Di potassium glycyrrhezinate 0.5 wt.% | -63 |
| + Ronacare® Ectoin 0.1 wt.% | -31 |
| + Ronacare® Ectoin 0.25 wt.% | -60 |
| + Willow herb 0.1 wt.% + Oat Straw 0.1 wt.% | -32 |

### Example 3

Based on the foregoing results, Dragocalm®, ectoine, and dipotassium glycyrrhenzinate were tested further on human volunteers for their potential to reduce retinol induced skin redness.

Patches containing formulas comprising either retinol (0.1 wt.%) or retinol (0.1 wt.%) plus the test ingredient Dragocalm®, ectoine, or dipotassium glycyrrhenzinate were applied during 48 hours to the backs of volunteers three times a week during three weeks. Redness induced by the patches was examined and scored after each patch removal. Every individual score for each product were cumulated to give the final scores.

The results are summarized in Table 2 below. Dragocalm® at a concentration of 3.5 wt.% was able to induce a marked inhibition of retinol-induced redness. Ectoine also reduced redness but to a lesser extent, whereas dipotassium glycyrrhenzinate was not effective.

**Table 3**

| | Score | % reduction |
|---|---|---|
| Control + Retinol 0.1 wt.% | 21,5 | |
| Control + Retinol 0.1 wt.% | | |
| + Dragocalm 3.5 wt.% | 6,5 | -70 |
| + Ectoin 0,25 wt.% | 9,5 | -56 |
| + Di potassium glycyrrhezinate 0.5 wt.% | 17 17 | -21 -21 |

### Example 4

Dragocalm® (3.5 wt.%) was introduced into a product containing retinol (0.1 wt.%) and given to a panel of consumers for in-use testing during six weeks. Adverse reactions observed with this composition, which was according to the invention, were compared with results obtained for a control composition not containing Dragocalm® but otherwise the same. Thirty-seven percent of the volunteers experienced adverse reactions with the control composition. However, only 19% of the volunteers using the composition of the invention experienced adverse reactions. This demonstrates that addition of Dragocalm® into a retinol-containing product decreased retinol-induced adverse reactions.

It is understood that while the invention has been described in conjunction with the detailed description thereof, that the foregoing description is intended to illustrate, and not limit the scope of the invention, which is defined by the scope of the appended claims. Other aspects, advantages, and modifications are within the claims.

## Claims

1. A composition comprising:
(a) at least one retinoid, and
(b) at least one interleukin-8 inhibitor,
(c) wherein said composition provides at least about a 40 percent reduction in interleukin-8 produced by human epidermis after contact with said composition compared with a control composition comprising said retinoid but not said interleukin-8 inhibitor.

2. The composition of claim 1, wherein said retinoid is selected from the group consisting of retinol, retinal, and retinoic acid.

3. The composition of claim 1, wherein said interleukin-8 inhibitor comprises an oat extract.

4. The composition of claim 3, wherein said oat extract is an avenanthramide.

5. The composition of claim 1, wherein said interleukin-8 inhibitor comprises ectoine or an ectoine derivative.

6. The composition of claim 1 comprising about 0.001 to about 20 weight percent of said retinoid.

7. The composition of claim 1 comprising about 0.0005 to about 5 weight percent of interleukin-8 inhibitor.

8. A composition comprising:
(a) about 0.001 to about 20 weight percent of at least one retinoid, in particular retinol, and
(b) about 0.0005 to about 5 weight percent of at least one interleukin-8 inhibitor.

9. The composition of claim 8, wherein said composition provides at least about a 40 percent reduction in interleukin-8 produced by human epidermis after contact with said composition compared with a control composition comprising said retinoid but not said interleukin-8 inhibitor.
